(19) Europäisches Patentamt / European Patent Office / Office européen des brevets

(11) **EP 4 691 439 A1**

(12) **EUROPEAN PATENT APPLICATION**
published in accordance with Art. 153(4) EPC

(43) Date of publication:
**11.02.2026 Bulletin 2026/07**

(21) Application number: **23930820.8**

(22) Date of filing: **10.11.2023**

(51) International Patent Classification (IPC):
*A61F 13/15* (2006.01)

(52) Cooperative Patent Classification (CPC):
**A61F 13/15**

(86) International application number:
**PCT/JP2023/040577**

(87) International publication number:
**WO 2024/202191 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.03.2023 JP 2023055944**

(71) Applicant: **DAIO PAPER CORPORATION
Shikokuchuo-shi
Ehime 799-0492 (JP)**

(72) Inventor: **ITO, Rina
Sakura-shi, Tochigi 329-1411 (JP)**

(74) Representative: **Epping - Hermann - Fischer
Patentanwaltsgesellschaft mbH
Schloßschmidstraße 5
80639 München (DE)**

(54) **WRAPPING SHEET, INDIVIDUALLY WRAPPED ABSORBENT ARTICLE FORMED BY INDIVIDUALLY WRAPPING ABSORBENT ARTICLE WITH SAME, AND METHOD FOR MANUFACTURING WRAPPING SHEET**

(57)     A wrapping sheet includes paper. A surface of the wrapping sheet is subjected to printing while the wrapping sheet is conveyed in a fiber direction of the wrapping sheet, and the wrapping sheet individually wraps an absorbent article. A tensile strength of the wrapping sheet in the fiber direction is 16 N or greater and 25 N or less. The wrapping sheet satisfies $8.45 \leq X \times (1 - Y/100)$, where $X$ (N) is the tensile strength of the wrapping sheet in the fiber direction before the printing, and $Y$ (%) is a ratio of an area of the wrapping sheet, to which an ink for the printing, a heat seal agent for adhering portions of the wrapping sheets, or both are applied, to an entire area of the wrapping sheet.

FIG.6

(a)

(b)

EP 4 691 439 A1

**Description**

TECHNICAL FIELD

The present invention relates to wrapping sheets, individually-wrapped absorbent articles each formed by individually wrapping an absorbent article with a wrapping sheet, and production methods of wrapping sheets.

BACKGROUND ART

From reasons of hygiene during storage, convenience when carrying, and the like, many absorbent articles, such as sanitary napkins, pantyliners, incontinence pads, and the like, are provided as individually-wrapped absorbent articles (individual packages) in the state in which an absorbent article is individually wrapped with a wrapping sheet and sealed.
Such an individually-wrapped absorbent article is carried in a state in which an absorbent article is wrapped with a wrapping sheet. Thus, it may be often preferable to print on a surface of the wrapping sheet so that an appearance of the individually-wrapped absorbent article is not apparent as an absorbent article at a glance.
Patent Document 1 discloses an absorbent article package that includes a absorbent article and a packaging material that packages the absorbent article, and has a design pattern that is visible from the outside.

RELATED-ART DOCUMENTS

PATENT DOCUMENTS

Patent Document 1: International Publication No. WO2020/095764

SUMMARY OF THE INVENTION

PROBLEM TO BE SOLVED BY THE INVENTION

In recent years, instead of conventional non-woven fabrics or plastic films, use of a paper material to form a wrapping sheet has been promoted in view of environmental issues. In the case where a wrapping sheet is made of a paper material, tearing of the wrapping sheet, i.e., tearing of paper, during printing or the like, needs to be avoided.
The present invention has been made in view of the above problems existing in the related art, and an object of the present invention is to provide a wrapping sheet that can avoid tearing when printing is performed on a surface of the wrapping sheet, an individually-wrapped absorbent article formed by individually wrapping an absorbent article with the wrapping sheet, and a production method of the wrapping sheet.

SOLUTION TO THE PROBLEM

In order to achieve the above object, the present invention is directed to a wrapping sheet that includes paper. A surface of the wrapping sheet is subjected to printing while the wrapping sheet is conveyed in a fiber direction of the wrapping sheet, and the wrapping sheet individually wraps an absorbent article. A tensile strength of the wrapping sheet in the fiber direction is 16 N or greater and 25 N or less. The wrapping sheet satisfies $8.45 \leq X \times (1 - Y/100)$, where X (N) is a tensile strength of the wrapping sheet in the fiber direction before the printing, and Y (%) is a ratio of an area of the wrapping sheet, to which an ink for the printing, a heat seal agent for adhering portions of the wrapping sheets, or both are applied, to an entire area of the wrapping sheet.
In the present invention having the above configuration, the tensile strength of the wrapping sheet in the conveying direction is 16 N or greater and 25 N or less, and the tensile strength of the wrapping sheet before the printing and the ratio of the area of the wrapping sheet to which an ink for the printing, an heat seal agent, or both are applied to the entire area of the wrapping sheet satisfy the above condition. Thus, tearing of the wrapping sheet can be avoided when the printing is performed on the surface of the wrapping sheet.
Moreover, the wrapping sheet may be configured such that MMD (deviation of the mean value of the coefficient of friction) of the surface of the wrapping sheet to be subjected to the printing is 0.050 or less.
The wrapping sheet having the above configuration allows uniform printing to be performed on the surface of the wrapping sheet, thereby improving the appearance.
Moreover, the wrapping sheet may be configured such that a paper basis weight of the wrapping sheet is 30 gsm or less.
The wrapping sheet having the above configuration can avoid problems such that a material cost increases due to a high paper basis weight, and operability is impaired and a noise is generated at the time of opening due to high stiffness of paper.

Moreover, an individually-wrapped absorbent article may be configured such that the individually-wrapped absorbent article includes the wrapping sheet and an absorbent article individually wrapped with the wrapping sheet, where the wrapping sheet is folded and edge portions of the folded wrapping sheet are sealed to individually package the absorbent article, and a seal strength of the edge portions is smaller than the tensile strength.

In the individually-wrapped absorbent article having the above configuration, the wrapping sheet is unlikely to be torn when the sealed portions are peeled off to take out the absorbent article.

Moreover, a production method of a wrapping sheet is a production method of a wrapping sheet that individually wraps an absorbent article. The production method includes at least one of: a printing step of printing on a surface of the wrapping sheet while conveying the wrapping sheet in a fiber direction of the wrapping sheet; and an application step of applying a heat seal agent on the surface of the wrapping sheet while conveying the wrapping sheet in the fiber direction. A tensile strength of the wrapping sheet in the fiber direction is 16 N or greater and 25 N or less. The wrapping sheet satisfies $8.45 \leq X \times (1 - Y/100)$, where $X$ (N) is a tensile strength of the wrapping sheet in the fiber direction before the printing, and $Y$ (%) is a ratio of an area of the wrapping sheet, to which an ink for the printing, a heat seal agent for adhering portions of the wrapping sheets, or both are applied, to an entire area of the wrapping sheet.

EFFECTS OF THE INVENTION

According to the present invention, tearing of a wrapping sheet can be avoided when printing is performed on a surface of the wrapping sheet.

BRIEF DESCRIPTION OF THE DRAWINGS

[Fig. 1] Fig. 1 is a plan view illustrating one embodiment of the individually-wrapped absorbent article of the present invention.
[Fig. 2] Fig. 2 is a plan view of a developed state of the individually-wrapped absorbent article illustrated in Fig. 1.
[Fig. 3] Fig. 3 is a cross-sectional view taken along the line I-I illustrated in Fig. 1.
[Fig. 4] Fig. 4 is an enlarged view of the section II illustrated in Fig. 1.
[Fig. 5] Fig. 5 is a view for explaining a method of printing on the wrapping sheet illustrated in Figs. 1 to 3.
[Fig. 6] Fig. 6 is a view illustrating a detailed configuration of a printed surface of the wrapping sheet illustrated in Figs. 1 to 3.

DESCRIPTION OF EMBODIMENTS

Embodiments of the present invention will be described with reference to drawings hereinafter. In the drawings, the same referential numeral is assigned to the same or corresponding constituent component unless otherwise stated, and redundant description may be omitted.

<Overall configuration of individually-wrapped absorbent article>

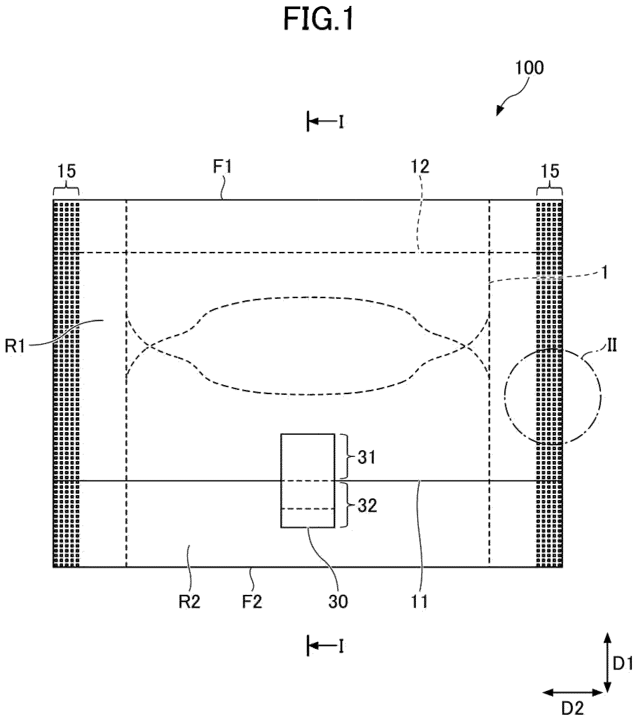

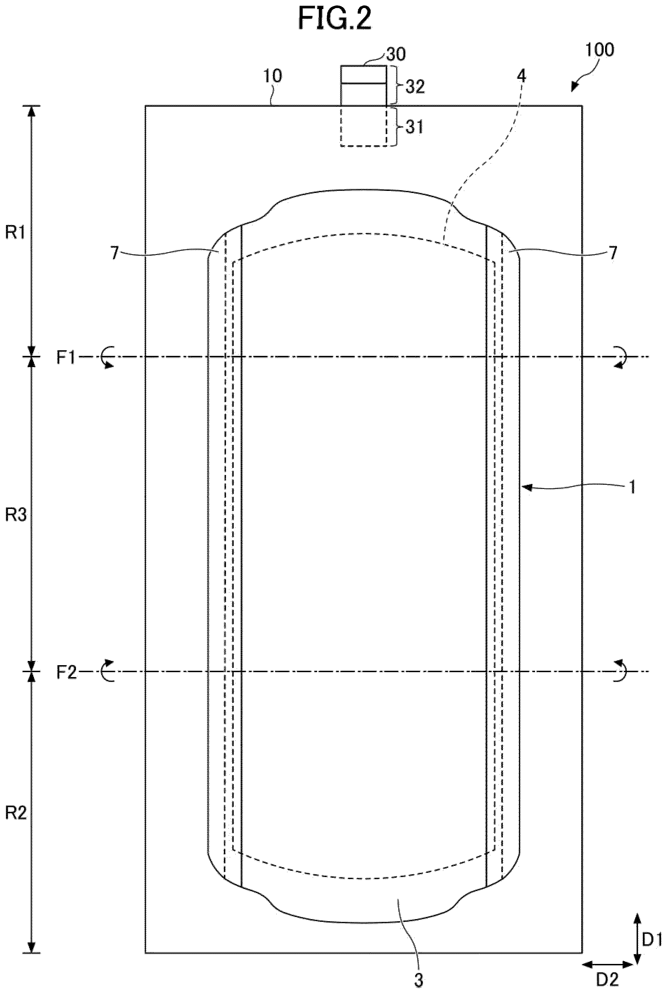

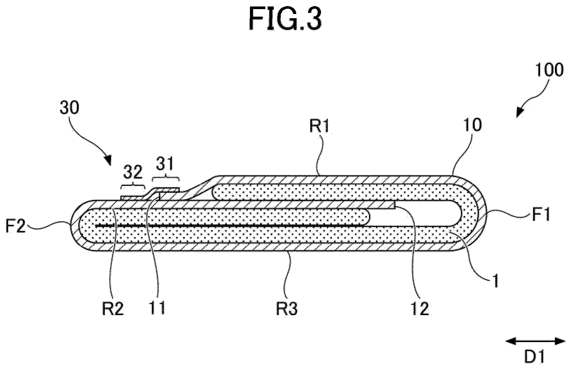

Fig. 1 is a plan view illustrating one embodiment of the individually-wrapped absorbent article of the present invention. Fig. 2 is a plan view of a developed state of the individually-wrapped absorbent article 100 illustrated in Fig. 1, and illustrates a view as viewed from the inner surface of the wrapping sheet 10 or the side of the absorbent article 1 facing the skin of a user. Moreover, Fig. 3 is a cross-sectional view taken along the line I-I illustrated in Fig. 1.

As illustrated in Figs. 1 to 3, the present embodiment is an individually-wrapped absorbent article 100 that includes a wrapping sheet 10, and an absorbent article 1 individually wrapped with the wrapping sheet 10. As illustrated in Fig. 2, the wrapping sheet 10 has, for example, a narrow and long shape in a developed state, and has a longitudinal direction (vertical direction) D1 and a lateral direction (horizontal direction) D2 orthogonal to the longitudinal direction. Moreover, the longitudinal direction D1 and the lateral direction D2 of the wrapping sheet 10 also correspond to a longitudinal direction and a lateral direction of the absorbent article 1, respectively.

<Absorbent article>

The absorbent article 1 to be wrapped with the wrapping sheet 10 is an article having a shape that is suitable when the article is worn to face a discharge orifice of a body fluid (menstrual blood, vaginal discharge, urine, etc.), for example, a flat, narrow, and long shape. Specific examples of the absorbent article 1 includes sanitary napkins, pantyliners (vaginal discharge sheets), light incontinence pads, and the like. In the present specification, the description is mainly given based on an example in which the absorbent article is a sanitary napkin.

The absorbent article 1 includes, for example, a fluid-permeable top sheet 3, a fluid-impermeable back sheet (not illustrated), and an absorber 4 disposed between the top sheet 3 and the back sheet, as illustrated in Fig. 2.

As the back sheet, a sheet having at least a water-shielding property, such as an olefin-based resin sheet or the like, may be used. The olefin-based resin is such as polyethylene, polypropylene, or the like. A laminate non-woven fabric, in which a non-woven fabric is laminated on a polyethylene sheet or the like, or a laminate sheet of non-woven fabrics, in which a waterproof film is inserted between the non-woven fabrics to substantially ensure a fluid impermeability, may be used as the back sheet. Further, a sheet having moisture permeability may be used as the back sheet.

As the top sheet 3, a porous or non-porous non-woven fabric, a porous plastic sheet, or the like is suitably used. As a fibrous material constituting the non-woven fabric, for example, one of, or two or more of synthetic fibers, such as olefin (e.g., polyethylene, polypropylene, etc.), polyester, polyamide, and the like, recycled fibers, such as rayon, cuprammonium rayon, and the like, blended fibers of the foregoing, and natural fibers, such as cotton and the like can be used.

The absorber 4 is not limited as long as the absorber 4 is a material that can absorb and retain a body fluid. The absorber 4 preferably includes cotton pulp and a water-absorbing polymer. As the water-absorbing polymer, a superabsorbent polymer (SAP) powder, a superabsorbent fiber (SAF), or a combination of the foregoing can be used. Examples of the pulp include chemical pulp obtained from wood, cellulose fibers, such as dissolving pulp, artificial cellulose fibers, such as rayon, acetate, and the like. The pulp may be hardwood pulp obtained from hardwood, softwood pulp obtained from softwood, or a pulp mixture of the foregoing. Moreover, the pulp may be recycled pulp that is recycled from used pulp.

A length of the absorber 4 may be from 0.5 mm to 25 mm. The absorber 4 may be configured such that a region corresponding to a body fluid discharge orifice (body-fluid-discharge-orifice corresponding region) or a region facing the intergluteal cleft behind the body-fluid-discharge-orifice corresponding region is bulged. The absorber 4 has a size and a shape that do not exceed the top sheet 3 and the back sheet. At the front and rear end edges of the absorber, the outer edges of the back sheet and the outer edges of the top sheet 3 are respectively joined by an adhesive such as a hot-melt adhesive or the like, or an adhesion method such as heat sealing or ultrasonic sealing.

As illustrated in Fig. 2, side sheets 7 may be respectively provided on the outer sides of the absorber 4 along the longitudinal direction D1 at the both edge portions of the absorber 4 in the lateral direction D2. As the side sheets 7, water-repellent non-woven fabrics or hydrophilic non-woven fabrics can be used.

Note that the absorbent article 1 is a so-called wingless type that does not have wings in the example illustrated in Figs. 1 to 3, but the absorbent article may be configured as an article having wings each extending to the side. The wings may be formed by joining the side sheet and the back sheet.

Moreover, an anti-slip adhesion portion may be provided on a back sheet side of the absorbent article 1 (the non-skin side, i.e., the side facing the underwear when worn). The anti-slip adhesion portion is provided for preventing the absorbent article 1 from being displaced from the underwear when the absorbent article 1 is attached to the underwear. The anti-slip adhesion portion is, for example, formed into multiple strips extending in the longitudinal direction D1 or the lateral direction D2.

An entire length of the absorbent article 1 can be from 140 mm to 430 mm, and a width of the absorbent article 1 (a width of a main body excluding wings if wings are provided) can be from 40 mm to 130 mm.

<Wrapping sheet>

The wrapping sheet 10 of the present embodiment includes paper. Use of the wrapping sheet 10 made of paper can contribute to reduction in use of plastics, and to the achievement of sustainable development goals. Moreover, paper can provide unique textures, such as the appearance and feel that give gentle impressions natural materials have.

The wrapping sheet 10 includes pulp fibers in an amount of 50% or greater, and a design print is applied to an exposed surface of the wrapping sheet that is exposed in a state in which the absorbent article 1 is individually wrapped with the wrapping sheet. The detailed conditions of the wrapping sheet 10 will be described later.

A size of the wrapping sheet 10 depends on a size or shape of an absorbent article 1 to be wrapped. For example, a length in the longitudinal direction D1 (may be merely referred to as a length) can be from 100 mm to 450 mm, and a length in the lateral direction D2 (may be merely referred to as a width) can be from 70 mm to 250 mm in the state in which the wrapping sheet 10 is completely extended (the unfolded state). In the illustrated example, the wrapping sheet 10 has a rectangular shape in a developed state, but may have, for example, a shape, such as an oblong shape.

<Package configuration of individually-wrapped absorbent article>

As illustrated in Figs. 1 to 3, the absorbent article 1 is wrapped by being folded together with the wrapping sheet 10, thereby forming an individually-wrapped absorbent article 100. At the time of folding, first, the absorbent article 1 is placed on the inner surface of the wrapping sheet 10 in the manner such that the back sheet side of the absorbent article 1 faces the inner surface of the wrapping sheet 10, as illustrated in Fig. 2. Then, the wrapping sheet 10 and the absorbent article 1 are folded together in the longitudinal direction D1 at the first folding line F1 and the second folding line F2 along the width direction D2. More specifically, a first region R1 including one end 11 of the wrapping sheet 10 in the longitudinal direction D1 is folded over in the longitudinal direction D1 along the first folding line F1, and a second region R2 including the other end 12 of the wrapping sheet 10 in the longitudinal direction D1 is folded over along the second folding line F2. A region of the wrapping sheet 10 between the first region R1 and the second region R2 is a third region R3. In the illustrated example, the wrapping sheet 10 is folded in a manner such that the second region R2 is folded first, and then the first region R1 is folded to overlap the outer surface of the second region R2 (Figs. 1 and 2), but the order of folding the first region R1 and the second region R2 may be reversed. After the wrapping sheet 10 and the absorbent article 1 are together folded over in three (inward tri-fold) to wrap the absorbent article 1, both edge portions in the lateral direction D2 are sealed along the longitudinal direction D1, thereby forming sealed portions 15.

Such a wrapping form of three fold or greater can be formed relatively simply, and can wrap an absorbent article 1 hygienically. In addition, the absorbent article 1 can be easily taken out. Note that the folding form is not limited to three fold, and may be four fold or more, or may be two fold.

<Sealed portion>

As illustrated in Fig. 1, portions of the individually-wrapped absorbent article 100 where the absorbent article 1 is not present at both edges in the lateral direction D2, are respectively sealed, thereby forming sealed portions 15. In the present embodiment, the sealed portions 15 are formed by applying a heat seal agent to the surface of the wrapping sheet 10 to be sealed, then passing through the wrapping sheet 10 between a pair of heated rolls to pressure bond the above overlapped portions of the folded wrapping sheet 10 in a thickness direction.

As illustrated in Fig. 1, the sealed portions 15 are formed, for example, along an entire length of the individually-wrapped absorbent article 100 in the longitudinal direction D1. This is preferable from the viewpoint that erroneous entry of dust or dirt, or a finger or a small object from the end edges in the lateral direction can be prevented (sealability is assured).

A range of the lateral direction D2 in which each sealed portion 15 is provided is, for example, a range of 20 mm from the end edge in the lateral direction D2. The sealed portion 15 may be formed in the entire range, or may be formed in part of the above range, for example, at a position away from the end edge in the lateral direction D2. A length (width) of each sealed portion 15 itself in the lateral direction D2 is preferably from 3 mm to 15 mm.

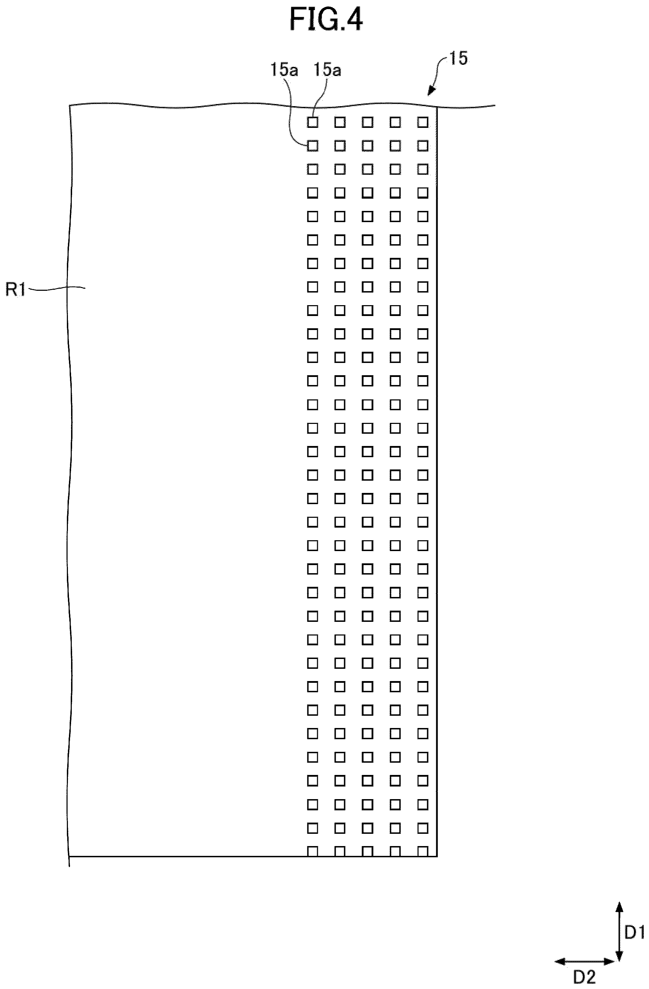

Fig. 4 is an enlarged view of the section II illustrated in Fig. 1, and illustrates a portion including the sealed portion 15.

As illustrated in Fig. 4, the sealed portion 15 includes multiple pressure-bonded portions (or bonded portions) 15a that are separated from one another in a plan view. Each pressure-bonded portion 15a is a portion in which overlapped portions of the folded wrapping sheet 10 are bonded by pressure bonding. In the illustrated example, a portion other than the pressure-bonded portions 15a is a non-bonded portion in which the overlapped portions of the folded wrapping sheet 10 are not bonded to each other. Since the pressure-bonded portions 15a are scattered apart from one another as described above, the bonded portions of the wrapping sheet can be easily peeled off at the time of opening, and openability can be improved, for example, as compared with a case where the portions of the wrapping sheets 10 are bonded over the entire surface of the sealed portion 15. Moreover, this can prevent the both edges of the individually-wrapped absorbent article 100 from becoming excessively hard and impairing the texture.

Note that a shape of each pressure-bonded portion 15a in a plan view is a square in the example illustrated in Fig. 4, but the shape is not limited to the illustrated shape and may be, for example, a quadrangle other than a square, such as a rectangle or a parallelogram, a polygon other than a quadrangle, a circle, an ellipse, a heart shape, a star shape, a drop shape, or the like. Moreover, a size of each pressure-bonded portion 15a is, for example, a square having a side of 0.25 mm to 2.5 mm or an equivalent size having the same area as the square. Further, an arrangement of the pressure-bonded portions 15a may be a lattice pattern as illustrated in Fig. 4, or may be a staggered pattern.

(Fastening tape)

As illustrated in Figs. 1 to 3, the individually-wrapped absorbent article 100 may be provided with a fastening tape 30 at a center in the lateral direction D2 in the vicinity of the edge end (one end 11 of the wrapping sheet 10). As illustrated in Fig. 1, the fastening tape 30 may be provided over the first region R1 and the second region R2 across one end 11 of the wrapping sheet 10. The fastening tape 30 includes a proximal portion 31 bonded to the first region R1, and a tip portion 32 bonded to the second region R2 in a plan view.

Since the fastening tape 30 is provided, a user can hold the fastening tape 30 (more specifically, the tip portion 32 of the fastening tape 30) to lift and pull the first region R1 at the time of opening the individually-wrapped absorbent article 100 so that the opening of the individually-wrapped absorbent article 100 is further facilitated. Moreover, when an absorbent article is replaced, the presence of the fastening tape 30 makes it possible to wrap a used absorbent article with a wrapping sheet obtained from a freshly opened individually-wrapped absorbent article, and fasten the end of the wrapping sheet with another part of the wrapping sheet. This can prevent the used absorbent article from being exposed due to the opening of the wrapping sheet, and can dispose of the used absorbent article hygienically.

A width (length in the lateral direction D2) of the fastening tape 30 may be preferably from 5 mm to 30 mm, and more preferably from 10 mm to 20 mm. Moreover, an entire length of the fastening tape 30 may be preferably from 10 mm to 50 mm, and more preferably from 15 mm to 40 mm.

<Strength of wrapping sheet and ratio of ink applied area>

When the individually-wrapped absorbent article 100 configured as described above is produced, first, printing is performed on the wrapping sheet 10.

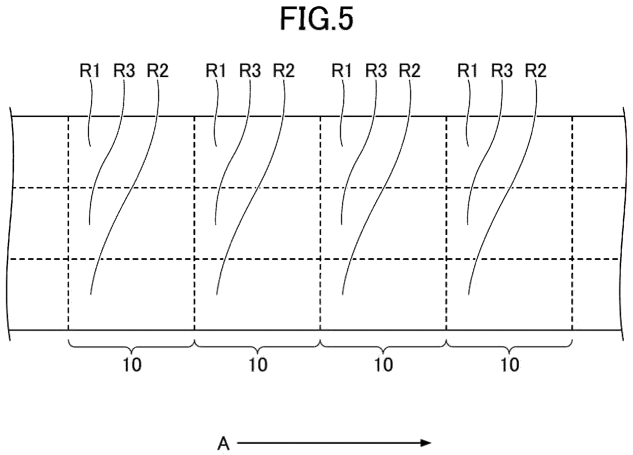

Fig. 5 is a view for explaining a method of printing on the wrapping sheet 10 illustrated in Figs. 1 to 3.

When the wrapping sheet 10 illustrated in Fig. 1 is produced, as illustrated in Fig. 5, in a printing step, printing is performed on surfaces of multiple wrapping sheets 10 in a state in which the wrapping sheets 10 are closely arranged next to one another in a fiber direction (machine direction (MD)) that is a lateral direction. At this time, printing is performed while conveying the wrapping sheets 10 in the lateral direction as a conveyance direction A. However, if the tensile strength of the wrapping sheet 10 is weak, the wrapping sheet is torn during printing or the like, that is, tearing of paper occurs.

Moreover, when printing is performed on the wrapping sheet 10, an ink used for the printing permeates the wrapping sheet 10, which loosens fibrous tissues, and weakens the inter-fiber bonding. This also reduces the tensile strength, thereby causing tearing of paper. The above phenomenon occurs not only with the ink of the printing. Even when the printing step is not provided, the above phenomenon also occurs due to application of a heat seal agent in an application step in which the heat seal agent is applied to the wrapping sheet 10 to seal the overlapped portions of the folded wrapping sheet 10.

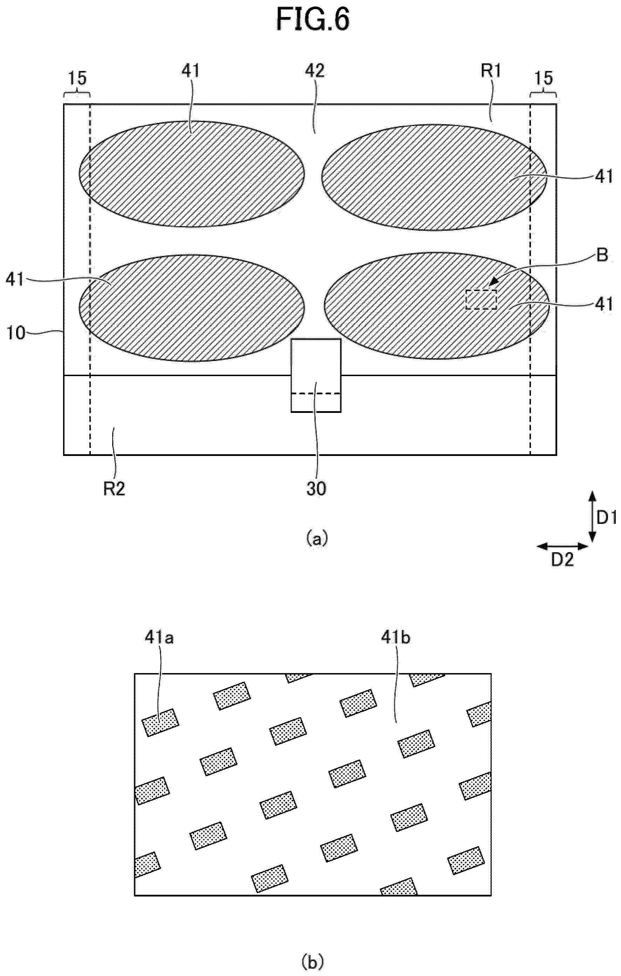

Fig. 6 includes views illustrating a printing example performed on the wrapping sheet 10 illustrated in Figs. 1 to 3, where (a) is a plan view of the individually-wrapped absorbent article, and (b) is an enlarged view of the section B in the case of dot printing.

When printing is performed on the wrapping sheet 10, as illustrated in Fig. 6(a), there are a colored regions 41 that are colored by printing, and a non-colored region 42 that is not printed. In the case of so-called solid printing performed by gravure printing, an ink is applied to the entire area of each colored region 41. Thus, a ratio of an area to which the ink is applied to the entire area of the wrapping sheet 10 is determined by:

(total area of colored regions 41)/(entire area of wrapping sheet 10)

On the other hand, in the case where printing is performed by dot printing, as illustrated in Fig. 6(b), there are applied regions 41a to which an ink is applied, and an unapplied region 41b to which the ink is not applied within each colored region 41 to which printing is performed. Thus, the ratio of the area to which the ink is applied to the entire area of the wrapping sheet 10 is determined by:

(total area of applied regions 41a)/{(area of non-colored region 42)+(total area of unapplied regions 41b)}.

That is, in the dot printing, the ratio of the area to which the ink is applied to the entire area of the wrapping sheet 10 can be set not to be 100% even when the entire area of the wrapping sheet 10 is subjected to printing.

Therefore, various types of paper that are considered to be usable as the wrapping sheet 10 were examined for printing operability based on a tensile strength, and a ratio of an area to which the ink or the heat seal agent is applied to the entire area of the wrapping sheet 10. Since printing was performed on the wrapping sheet 10 of the present embodiment while conveying the wrapping sheet 10 in the fiber direction of the paper, as the tensile strength, the tensile strength of the wrapping sheet 10 in the fiber direction was measured. In this examination, the measurement was performed at room temperature by a tensile tester, Tensilon. As the measurement conditions, a paper base material to be a wrapping sheet 10 was cut into an individual piece having a size of 120 mm in the fiber direction and 25 mm in the direction orthogonal to the fiber direction, and the tensile strength was measured while pulling the piece in the fiber direction at the speed of 500 mm/sec. The interval between the chucks was set to be 50 mm.

[Table 1]

| Sample | Ex. 1 | Ex. 2 | Ex. 3 | Ex. 4 | Ex. 5 | Comp. Ex. 1 | Comp. Ex. 2 | Comp. Ex. 3 | Comp. Ex. 4 | Comp. Ex. 5 |
|---|---|---|---|---|---|---|---|---|---|---|
| Paper type / Basis weight | Unryu-shi 14 gsm | Unryu-shi 14 gsm | Crepe paper 18 gsm | Crepe paper 18 gsm | Crepe paper 18 gsm | Unryu-shi 17 gsm | Rayon PM 14 gsm | Unryu-shi 25 gsm | Kin Shachi-hoko 24.4 gsm | Kin Shachi-hoko 30 gsm |
| Tensile strength in MD (N) | 16.9 | 16.9 | 17.7 | 17.7 | 17.7 | 25.7 | 26.8 | 53.9 | 38.6 | 30 |
| Usability | A | A | A | A | A | A | A | C | B | C |
| Printing operability | A | A | C | A | A | B | A | - | A | A |
| Heat seal agent area ratio (%) | 0 | 50 | 100 | 0 | 0 | 100 | 100 | Not performed | 100 | 100 |
| Ink area ratio (%) | 20 | 0 | 0 | 12 | 35 | 72 | 72 | Not performed | 72 | 72 |

As presented in Table 1, as Example 1, a wrapping sheet 10 made of Unryu-sgi (produced by Marubushi Paper Tec. Corporation) having a basis weight of 14 gsm, which was not coated with a heat seal agent, and had an ink applied area ratio of 20% relative to the entire area of the wrapping sheet 10, was used. As Example 2, a wrapping sheet 10 made of Unryu-shi (produced by Marubishi Paper Tec. Corporation) having a basis weight of 14 gsm, which was not coated with an ink, and had a heat seal agent applied area ratio of 50% relative to the entire area of the wrapping sheet 10, was used. As Example 3, a wrapping sheet 10 made of crepe paper (produced by Daio Paper Corporation) having a basis weight of 18 gsm, which was not coated with an ink, and had a heat seal agent applied area ratio of 100% relative to the entire area of the wrapping sheet 10, was used. As Example 4, a wrapping sheet 10 made of crepe paper (produced by Daio Paper Corporation) having a basis weight of 18 gsm, which was not coated with a heat seal agent, and an ink applied area ratio of 12% relative to the entire area of the wrapping sheet 10, was used. As Example 5, a wrapping sheet 10 made of crepe paper (produced by Daio Paper Corporation) having a basis weight of 18 gsm, which was not coated with a heat seal agent, and an ink applied area ratio of 35% relative to the entire area of the wrapping sheet 10, was used. As Comparative Example 1, a wrapping sheet 10 made of Unryu-shi (produced by Marubishi Paper Tec. Corporation) having a basis weight of 17 gsm, which had a heat seal agent applied area of 100% relative to the entire area of the wrapping sheet 10, and an ink applied area ratio of 72% relative to the entire area of the wrapping sheet 10, was used. As Comparative Example 2, a wrapping sheet 10 made of Rayon PM (produced by Marubishi Paper Tec. Corporation) having a basis weight of 14 gsm, which had a heat seal agent applied area of 100% relative to the entire area of the wrapping sheet 10, and an ink applied area ratio of 72% relative to the entire area of the wrapping sheet 10, was used. As Comparative Example 3, Unryu-shi (produced by Marubishi Paper Tec. Corporation) having a basis weight of 25 gsm was used. As Comparative Example 4, a wrapping sheet 10 made of Kin Shachihoko Paper (produced by Daio Paper Corporation) having a basis weight of 24.4 gsm, which had a heat seal agent applied area ratio of 100% relative to the entire area of the wrapping sheet 10, and an ink applied area ratio of 72% relative to the entire area of the wrapping sheet 10, was used. As Comparative Example 5, a wrapping sheet 10 made of Kin Shachihoko Paper (produced by Daio Paper Corporation) having a basis weight of 30 gsm, which had a heat seal agent applied area ratio of 100% relative to the entire area of the wrapping sheet 10, and an ink applied area ratio of 72% relative to the total area of the wrapping sheet 10, was used. Note that Rayon PM is a paper composed of 15% of rayon and 85% of pulp.

In Table 1, as the printing operability, "A" is given to the case where printing could be performed without tearing paper, and "C" is given to the case where the paper was torn and printing could not be performed. Moreover, as usability, whether or not there was a problem in use, such as edges of an individual package becoming sharp or a rustling sound being generated due to the paper becoming hard, was determined. Then, "A" is given to the case where there was no problem in usability, "C" is given to the case where an individual package is hard and the sound is generated during use, and "B is given to the case where there was a problem in either the hardness or the sound.

As a result, in Comparative Examples 3 and 5, the edges of the individual package became sharp, and the noticeable rustling sound was generated during use. It has been found from the above results that the edges of the individual package become sharp and noticeable rustling sound is generated during use when the tensile strength of the wrapping sheet is greater than 54 N. In addition, tearing of paper occurred during printing on the paper having the tensile strength of 17.7 N used in Examples 3 to 5. On the other hand, no tearing of paper occurred in Comparative Example 1 using the paper having the tensile strength of 25.7 N. It has been found from the above results that tearing of paper may occur during printing when the tensile strength of the wrapping sheet is 25 N or lower.

Therefore, the ratio of the area on which the ink or the heat seal agent was applied to the entire area of the wrapping sheet 10 was checked for the wrapping sheet having the tensile strength of 25 N or less. As a result, tearing of paper occurred during printing in Example 3 in which the wrapping sheet made of the paper having the tensile strength of 17.7 N had the heat seal agent applied area ratio of 100% relative to the entire area of the wrapping sheet 10. On the other hand, tearing of paper did not occur during printing in Example 5 in which the wrapping sheet made of the paper having the tensile strength of 17.7 N had the ink applied area ratio of 35% relative to the entire area of the wrapping sheet 10.

Moreover, tearing of paper did not occur during printing in Example 2 in which the wrapping sheet made of the paper having the tensile strength of 16.9 N had the heat seal applied area ratio of 50% relative to the entire area of the wrapping sheet 10 of the ink.

Here, the ink applied area ratio that enables printing is relatively large with respect to the strength of paper. Specifically, as the tensile strength of paper increases, the ink applied area ratio that enables printing increases. Therefore, the threshold value for enabling the printing, which is determined by (tensile strength) $\times$ (1 - ratio of area to which ink (heat seal agent) is applied), is a constant value.

Therefore, based on the above results, for the paper used in Example 2, which had the lowest tensile strength among the types of the paper examined as above, the heat seal applied area ratio of 50%, at which the printing operability was "A," was assigned in the above formula, and 16.9 N was assigned as the tensile strength, thereby leading to the value, which is $16.9 \times (1 - 0.50) = 8.45$. Then, this value is set as the lower limit so that tearing of paper will not occur during printing.

Thus, it has been found that a wrapping sheet satisfying the relationship of $8.45 \leq X \times (1 - Y/100)$ does not cause tearing of paper during printing, where X (N) is the tensile strength, and Y (%) is a ratio of the area to which the ink, the heat seal agent, or both are applied to the entire area of the wrapping sheet. Note that the above formula stands in any of cases where any of the values, with which the printing operability was "A," are assigned to the algebra of X and Y.

Note that Unryu-shi, Rayon PM, and Kin Shachihoko had printing operability even when the heat seal agent area ratio was 100%. Therefore, the restriction on the applied area is not needed for the paper having the tensile strength of greater than 25 N. In addition, the paper having the tensile strength of less than 16 N has insufficient printing operability regardless of an ink or heat seal agent applied area ratio, and is likely to cause breakage of an individual package during opening.

It was found from the above results that, when the wrapping sheet 10 that has the tensile strength of 16 N or greater and 25 N or less in the conveying direction during printing, and satisfies $8.45 \leq X \times (1 - Y/100)$, where X (N) is the tensile strength before printing, and Y (%) is the ratio of the area to which the ink for the printing or the heat seal agent for adhering overlapped portions of the folded wrapping sheet to the entire area of the wrapping sheet 10, is used, tearing can be avoided when printing is performed on the surface of the wrapping sheet. In addition, it is also possible to avoid edges of the individual package from becoming sharp and generation of noticeable rustling sound during use.

Thus, it is possible to provide an individually-wrapped absorbent article 100 using the wrapping sheet 10 made of paper, which can be printed using thin paper as the wrapping sheet 10 and has high designability, while ensuring operability and usability.

In this case, if the surface of the wrapping sheet 10 is rough, printing is not uniformly performed, which impairs the appearance. Therefore, an experiment for determining whether printability was high was conducted by measuring MMD (deviation of the mean value of the coefficient of friction), which is an index indicating the smoothness of the paper surface. As a result, it was found that the paper had the printability when the surface of the paper had MMD of 0.050 or less before printing, and use of such paper allowed printing to be performed on the wrapping sheet 10, thereby improving the design.

Further, if the paper basis weight is high, the material cost increases. In addition, the stiffness of the paper is increased, which causes problems such as poor operability and noise during opening. Therefore, the paper basis weight is preferably 30 gsm or less.

<Sealed portion strength>

As described above, in the individually-wrapped absorbent article 100, sealed portions 15 are formed by folding over the wrapping sheet 10 together with the absorbent article 1 in three (inward tri-fold) to wrap the absorbent article 1, and sealing both edge portions of the folded wrapping sheet 10 in the lateral direction D2 along the longitudinal direction D1. Then, the sealed portions 15 that bond the overlapped portions of the folded wrapping sheet 10 are peeled so that the individually-wrapped absorbent article 100 is opened and the absorbent article 1 is taken out.

If the seal strength of the sealed portions 15 is high, the wrapping sheet 10 may be torn when the sealed portions 15 that bond the overlapped portions of the folded wrapping sheet 10 are peeled in order to take out the absorbent article 1.

Therefore, a relationship between tensile strength and the seal strength of the sealed portion was examined for the configuration in which the wrapping sheet 10 was not torn when the overlapped portions of the folded wrapping sheet 10 were peeled. In this examination, the measurement was performed at room temperature by a tensile tester, Tensilon. As the measurement conditions, the wrapping sheet 10 was cut into a piece having a width of 25 mm to include the sealed portion 15 (seal width of 6 mm) in the longitudinal direction of the wrapping sheet, and the tensile strength was measured while pulling the sealed portion of the cut sample in the longitudinal direction of the wrapping sheet 10. For the measurement, the pulling speed was set to be 100 mm/sec, and the interval between the chucks was set to be 10 mm. The maximum point load (N/measured width) was determined as the strength of the sealed portion 15.

[Table 2]

| Sample | Ex. 1 | Comp. Ex. 1 | Comp. Ex. 2 |
|---|---|---|---|
| Type of paper<br>Basis weight | Crepe paper<br>18 gsm | Unryu-shi<br>17 gsm | Rayon PM<br>14 gsm |
| Tensile strength after printing (N/mm) in MD | 0.60 | 1.55 | 1.11 |
| in CD | 0.19 | 0.39 | 0.32 |
| Seal strength of individual package (N/mm) | 0.03 | 0.06 | 0.04 |

As presented in Table 2, crepe paper (produced by Daio Paper Corporation) having a basis weight of 18 gsm was used as Example 1. As Comparative Example 1, Unryu-shi (produced by Marubishi Paper Tec. Corporation) having a basis weight of 17 gsm was used. As Comparative Example 2, Rayon PM (produced by Marubishi Paper Tec. Corporation) having a basis weight of 14 gsm was used. As a result of the examination on the above paper, the paper of Example 1 and the paper of Comparative Examples 1 and 2 had the seal strength of the sealed portion 15 that was smaller than the tensile strength of the wrapping sheet 10.

It has been found from the above that, if the seal strength of the sealed portion 15 is smaller than the tensile strength of the wrapping sheet 10, the wrapping sheet 10 is unlikely to be torn when the sealed portions 15 that bond the overlapped portions of the folded wrapping sheet 10 are peeled off in order to take out the absorbent article 1.

Although the present invention has been described through the embodiments above, the present invention is not limited to the above embodiments. The above embodiments can be varied by changing, modifying, replacing, adding, deleting, combining, or the like in various manners within the scope described in the claims, and such embodiments also belong within the technical scope of the present invention. Moreover, the above-described constituent components can be arbitrarily combined.

This international application claims priority based on the Japanese patent application 2023-055944 filed on March 30, 2023, the entire contents of which are incorporated herein by reference.

DESCRIPTION OF THE REFERENCE NUMERALS

1 absorbent article
3 top sheet
4 absorber
7 side sheet
10 wrapping sheet
11 one end in the longitudinal direction
12 the other end in the longitudinal direction
15 sealed portion
15a pressure-bonded portion
30 fastening tape
31 proximal portion
32 tip portion
41 colored region
41a applied region
41b unapplied region
42 non-colored region
100 individually-wrapped absorbent article

D1 longitudinal direction of wrapping sheet
D2 lateral direction of wrapping sheet
F1 first folding line
F2 second folding line
R1 first region
R2 second region
R3 third region

**Claims**

1. A wrapping sheet comprising:
   paper,

   wherein a surface of the wrapping sheet is subjected to printing while the wrapping sheet is conveyed in a fiber direction of the wrapping sheet, and the wrapping sheet individually wraps an absorbent article,
   wherein a tensile strength of the wrapping sheet in the fiber direction is 16 N or greater and 25 N or less, and
   wherein the wrapping sheet satisfies:

$$8.45 \leq X \times (1 - Y/100),$$

   where X (N) is the tensile strength of the wrapping sheet in the fiber direction before the printing, and Y (%) is a ratio of an area of the wrapping sheet, to which an ink for the printing, a heat seal agent for adhering portions of the wrapping sheets, or both are applied, to an entire area of the wrapping sheet.

2. The wrapping sheet according to claim 1,
   wherein MMD of the surface of the wrapping sheet to be subjected to the printing is 0.050 or less.

3. The wrapping sheet according to claim 1 or 2,
   wherein a paper basis weight of the wrapping sheet is 30 gsm or less.

4. An individually-wrapped absorbent article, comprising:

   the wrapping sheet of claim 1 or 2; and
   an absorbent article individually wrapped with the wrapping sheet,
   wherein the wrapping sheet is configured such that the wrapping sheet is folded and edge portions of the folded wrapping sheet are sealed to individually package the absorbent article, and
   wherein a seal strength of the edge portions is smaller than the tensile strength.

5. A production method of a wrapping sheet that individually wraps an absorbent article, the production method comprising at least one of:

   a printing step of printing on a surface of the wrapping sheet while conveying the wrapping sheet in a fiber direction; and
   an application step of applying a heat seal agent on the surface of the wrapping sheet while conveying the wrapping sheet in the fiber direction,
   wherein a tensile strength of the wrapping sheet in the fiber direction is 16 N or greater and 25 N or less, and
   wherein the wrapping sheet satisfies:

$$8.45 \leq X \times (1 - Y/100),$$

   where X (N) is the tensile strength of the wrapping sheet in the fiber direction before the printing, and Y (%) is a ratio of an area of the wrapping sheet, to which an ink for the printing, a heat seal agent for adhering portions of the wrapping sheets, or both are applied, to an entire area of the wrapping sheet.

# FIG.1

# FIG.2

# FIG.3

# FIG.4

# FIG.5

R1 R3 R2  R1 R3 R2  R1 R3 R2  R1 R3 R2

10  10  10  10

A →

# FIG.6

(a)

(b)

## INTERNATIONAL SEARCH REPORT

| International application No. |
| --- |
| **PCT/JP2023/040577** |

| A. | CLASSIFICATION OF SUBJECT MATTER |
| --- | --- |

*A61F 13/15*(2006.01)i
FI: A61F13/15 220; A61F13/15 355A; A61F13/15 358

According to International Patent Classification (IPC) or to both national classification and IPC

| B. | FIELDS SEARCHED |
| --- | --- |

Minimum documentation searched (classification system followed by classification symbols)

A61F13/15

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | JP 2023-18807 A (DAIO PAPER CORPORATION) 09 February 2023 (2023-02-09) paragraphs [0031]-[0034], [0044]-[0050], [0067], fig. 1-3, 6 | 1-5 |
| Y | JP 2009-13521 A (UNI-CHARM CO., LTD.) 22 January 2009 (2009-01-22) paragraphs [0031]-[0053], table 1 | 1-5 |
| Y | JP 2007-282918 A (UNI-CHARM CO., LTD.) 01 November 2007 (2007-11-01) paragraphs [0053]-[0055] | 2-4 |
| Y | JP 8-324635 A (KAO CORPORATION) 10 December 1996 (1996-12-10) paragraphs [0022]-[0025] | 4 |
| A | JP 2022-24624 A (DAIO PAPER CORPORATION) 09 February 2022 (2022-02-09) entire text, all drawings | 1-5 |
| A | US 2023/0064927 A1 (Drylock Technologies NV) 02 March 2023 (2023-03-02) entire text, all drawings | 1-5 |
| A | JP 2022-127724 A (DAIO PAPER CORPORATION) 01 September 2022 (2022-09-01) paragraph [0056] | 1,5 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **30 November 2023** | **12 December 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/040577**

| Patent document cited in search report | | | Publication date (day/month/year) | Patent family member(s) | | | Publication date (day/month/year) |
|---|---|---|---|---|---|---|---|
| JP | 2023-18807 | A | 09 February 2023 | (Family: none) | | | |
| JP | 2009-13521 | A | 22 January 2009 | US paragraphs [0024]-[0046], table 1 | 2012/0048916 | A1 | |
| | | | | EP | 2163378 | A1 | |
| JP | 2007-282918 | A | 01 November 2007 | US paragraphs [0093]-[0094] | 2007/0244454 | A1 | |
| | | | | EP | 2008628 | A1 | |
| JP | 8-324635 | A | 10 December 1996 | (Family: none) | | | |
| JP | 2022-24624 | A | 09 February 2022 | US | 2023/0060828 | A1 | |
| | | | | EP | 4190289 | A1 | |
| US | 2023/0064927 | A1 | 02 March 2023 | EP | 3865421 | A1 | |
| JP | 2022-127724 | A | 01 September 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020095764 A

- JP 2023055944 A